# EUROPEAN PATENT SPECIFICATION

(11) **EP 3 950 932 B1**
(45) Date of publication and mention of the grant of the patent: **05.06.2024**
(21) Application number: 20783163.7
(22) Date of filing: 27.03.2020
(51) Int. Cl.: C12N 5/071, C12N 5/0735, C12N 5/077, C12N 5/0793, C12N 5/10, C12N 15/12, C12Q 1/6837, C12Q 1/6851, C12Q 1/6881

(54) **METHOD FOR PRODUCING PLURIPOTENT STEM CELLS CAPABLE OF DIFFERENTIATING INTO SPECIFIC CELLS, AND APPLICATION THEREOF**
VERFAHREN ZUR HERSTELLUNG PLURIPOTENTER STAMMZELLEN MIT FÄHIGKEIT ZUR DIFFERENZIERUNG IN SPEZIFISCHE ZELLEN UND DEREN VERWENDUNG
PROCÉDÉ DE PRODUCTION DE CELLULES SOUCHES PLURIPOTENTES CAPABLES DE SE DIFFÉRENCIER EN CELLULES SPÉCIFIQUES, ET APPLICATION ASSOCIÉE

(30) Priority: 29.03.2019 JP 2019065293
(43) Date of publication of application: 09.02.2022
(73) Proprietor: FUJIFILM Corporation, Tokyo 106-8620 (JP)
(72) Inventor: MURAKAMI, Yuta, Kanagawa 258-8577 (JP); NAGASE, Masaya, Kanagawa 258-8577 (JP)
(74) Representative: Hoffmann Eitle
(86) International application number: PCT/JP2020/013881
(87) International publication number: WO 2020/203712

(56) References cited:
- EP-A1- 2 374 871
- WO-A-2014/200030
- WO-A1-2012/029316
- WO-A1-2013/075222
- WO-A1-2014/200030
- WO-A1-2015/134652
- WO-A1-2016/148253
- WO-A2-2014/200905
- JP-A- 2013 528 397
- SMITH J R ET AL: "Inhibition of Activin/Nodal signaling promotes specification of human embryonic stem cells into neuroectoderm", DEVELOPMENTAL BIOLOGY, ELSEVIER, AMSTERDAM, NL, vol. 313, no. 1, 1 January 2008 (2008-01-01), pages 107-117, XP025612042, ISSN: 0012-1606, DOI: 10.1016/J.YDBIO.2007.10.003 [retrieved on 2007-10-11]
- DAE-SUNG KIM ET AL: "Robust Enhancement of Neural Differentiation from Human ES and iPS Cells Regardless of their Innate Difference in Differentiation Propensity", STEM CELL REVIEWS AND REPORTS, vol. 6, no. 2, 1 June 2010 (2010-06-01), pages 270-281, XP055007831, ISSN: 1550-8943, DOI: 10.1007/s12015-010-9138-1
- Y. S. CHEN ET AL: "Small Molecule Mesengenic Induction of Human Induced Pluripotent Stem Cells to Generate Mesenchymal Stem/Stromal Cells", STEM CELLS TRANSLATIONAL MEDICINE, vol. 1, no. 2, 1 February 2012 (2012-02-01), pages 83-95, XP055081405, ISSN: 2157-6564, DOI: 10.5966/sctm.2011-0022
- MCNAMARA H.M. et al.: "Geometry-dependent arrhythmias in electrically excitable tissues", Cell Systems, vol. 7, 2018, pages 359-370, XP055746981, ISSN: 2405-4712

## Description

### Technical Field

The present invention is defined in the appended claims and relates to a producing method for a pluripotent stem cell capable of differentiating into a specific cell. The present invention relates to a producing method for a differentiated cell. The present invention also relates to a method for quality evaluation of a pluripotent stem cell. Further, the present invention relates to a method for screening a pluripotent stem cell capable of differentiating into a specific cell.

### Background Art

In the field of regenerative medicine, research for inducing differentiation of a pluripotent stem cell into a specific cell is underway to obtain various differentiated cells.

The pluripotent stem cell is defined as a cell that has both the ability (the differentiation pluripotency) to differentiate into all cells that constitute a living body and the self-replication ability. However, in reality, it is known that the differentiation potency is not constant between donors from which pluripotent stem cells are derived or between established strains and that there are a cell having high differentiation potency and a cell having low differentiation potency.

Patent Document 1 disclose a method for screening an induced pluripotent stem (iPS) cell having high differentiation potency into the blood cell, where method including the following steps: (i) a step of measuring an expression level of one or more genes selected from the group consisting of TRIM58, CTSF, FAM19A5, and TCERG1L genes in an induced pluripotent stem cell as a sample; and (ii) a step of comparing a measured expression level of the gene above with an iPS cell or an embryonic stem (ES)cell, which is known to have high differentiation potency into the blood cell and selecting an induced pluripotent stem cell in which the measured expression level of the gene is comparable to or higher than that in the iPS cell or an embryonic stem (ES) cell, and/or a step of comparing a measured expression level of the gene above with an iPS cell or an ES cell, which is known to have low differentiation potency into the blood cell, and selecting an induced pluripotent stem cell in which the measured expression level of the gene is high.

### Prior Art Documents

### Patent Documents

Patent Document 1: WO2014/200030A
WO 2014/200905 A2 describes a set of early developmental reference data or "lineage scorecard" for stem cells, and methods, systems and kits to generate a lineage scorecard for predicting the functionality and suitability of stem cell lines.
WO 2014/200030 A1 describes a method for manufacturing hematopoietic stem cells and/or hematopoietic precursor cells from pluripotent stem cells, the method comprising a process for culturing the pluripotent stem cells in the presence of IGF2, and a method for selecting induced pluripotent stem cells having a high capacity for differentiating into hematopoietic stem cells and/or hematopoietic precursor cells or blood cells on the basis of the level of expression of one or more genes selected from a group consisting of TRIM58, CTSF, FAM19A5 and TCERG1L genes, or the DNA methylation state of the TRIM58, CSMD1 and/or FAM19A5 genes.
WO 2012/029316 A1 describes a method for screening for a differentiation-responsive induced pluripotent stem cell in a pluripotent stem cell sample, comprising measuring at least one of expression levels of H19 and/or Tnnt3 genes in the sample, or DNA methylation of H19 and/or Tnnt3 gene expression regulating regions in the sample, thus screening differentiation-responsive cell based on its property.

### SUMMARY OF THE INVENTION

A differentiated cell is obtained by stepwise differentiating a cell by culturing for a relatively long period of time using complicated culture conditions, and thus, in order to efficiently produce a differentiated cell product, it is required to select a pluripotent stem cell having high differentiation potency before differentiation induction and use it as a cell material. However, since the method disclosed in WO2014/200030A is a method for screening an iPS cell having high differentiation potency into the blood cell, a method for evaluating the differentiation potency into various other specific cells is required.

An object to be solved by the present invention is to provide a producing method for a pluripotent stem cell capable of differentiating into a specific cell. Another object to be solved by the present invention is to provide a pluripotent stem cell capable of differentiating into a specific cell obtained by the producing method for a pluripotent stem cell capable of differentiating into a specific cell. Another object to be solved by the present invention is to provide a producing method for a differentiated cell. Another object to be solved by the present invention is to provide a differentiated cell obtained by the above producing method for a differentiated cell. Further, another object to be solved by the present invention is to provide a method for quality evaluation of a pluripotent stem cell.

Under the above problems, as a result of detailed studies, the inventors of the present invention revealed that pluripotent stem cells can be classified into a strain in which a gene, which is expressed by the primitive endoderm cell, is expressed low and a strain in which the gene is expressed high, and the high expression strain has a low efficiency of differentiation into a specific cell. From this result, the inventors of the present invention have found that a pluripotent stem cell capable of differentiating into a specific cell can be screened by using an expression level of the gene expressed by the primitive endoderm cell as an indicator. The present invention has been completed based on the above findings.

That is, according to the present invention, the following inventions are provided.
1. A producing method for a pluripotent stem cell capable of differentiating into a specific cell, the method comprising:
   (i) a step of measuring an expression level of a gene, which is expressed by a primitive endodermal cell, in a pluripotent stem cell as a sample; and
   (ii) a step of comparing the measured expression level of the gene, which is expressed by the primitive endodermal cell, with an expression level of the gene in a pluripotent stem cell known to have high differentiation potency into the specific cell, and selecting a pluripotent stem cell in which the measured expression level of the gene is less than one time relative to the expression level in the pluripotent stem cell known to have high differentiation potency into the specific cell, and acquiring a pluripotent stem cell capable of differentiating into the specific cell,

   wherein the gene expressed by the primitive endodermal cell is at least one gene selected from the group consisting of CER1, LEFTY1, LEFTY2, NODAL, and CXCR4, and
   wherein the specific cell is a differentiated cell derived from mesoderm or ectoderm.
2. The method according to item 1,
   wherein the specific cell is a myocardial cell or a nerve cell.
3. The method according to item 1 or 2,
   wherein the pluripotent stem cell is a human derived cell.
4. The method according to any one of items 1 to 3,
   wherein the pluripotent stem cell as the sample is obtained by clonally culturing and proliferating a pluripotent stem cell.
5. A producing method for a differentiated cell, comprising:
   a step of obtaining a pluripotent stem cell capable of differentiating into a specific cell, by the method according to any one of items 1 to 4; and
   a step of inducing differentiation of the pluripotent stem cell capable of differentiating into a specific cell, obtained in the above step.
6. The method according to item 5,
   wherein the differentiated cell is a cell derived from mesoderm or ectoderm.
7. A method for quality evaluation of a pluripotent stem cell, the method comprising:
   (a) a step of measuring an expression level of a gene, which is expressed by a primitive endodermal cell, in a pluripotent stem cell as a sample;
   (b) a step of comparing the measured expression level of the gene, which is expressed by the primitive endodermal cell, with an expression level of the gene in a pluripotent stem cell known to have high differentiation potency into the specific cell; and
   (c) a step of determining that a pluripotent stem cell in which the measured expression level of the gene is less than one time relative to the expression level in the pluripotent stem cell known to have high differentiation potency into the specific cell is excellent,

   wherein the gene expressed by the primitive endodermal cell is at least one gene selected from the group consisting of CER1, LEFTY1, LEFTY2, NODAL, and CXCR4, and
   wherein the specific cell is a differentiated cell derived from mesoderm or ectoderm.
8. A method for screening a pluripotent stem cell capable of differentiating into a specific cell, the method comprising the following steps:
   (i) a step of measuring an expression level of a gene, which is expressed by a primitive endodermal cell, in a pluripotent stem cell as a sample; and
   (ii) a step of comparing the measured expression level of the gene, which is expressed by the primitive endodermal cell, with an expression level of the gene in a pluripotent stem cell known to have high differentiation potency into the specific cell, and selecting a pluripotent stem cell in which the measured expression level of the gene is less than one time relative to the expression level in the pluripotent stem cell known to have high differentiation potency into the specific cell,

   wherein the gene expressed by the primitive endodermal cell is at least one gene selected from the group consisting of CER1, LEFTY1, LEFTY2, NODAL, and CXCR4, and
   wherein the specific cell is a differentiated cell derived from mesoderm or ectoderm.

According to the present invention as defined in the appended claims, it is possible to provide a producing method for a pluripotent stem cell capable of differentiating into a specific cell. According to the present invention as defined in the appended claims, it is possible to provide a producing method for a differentiated cell. According to the present invention as defined in the appended claims, it is possible to provide a differentiated cell. According to the present invention as defined in the appended claims, it is possible to provide a method for quality evaluation of a pluripotent stem cell. Further, according to the present invention as defined in the appended claims, it is possible to provide a method for screening a pluripotent stem cell capable of differentiating into a specific cell.

### BRIEF DESCRIPTION OF THE DRAWINGS

Fig. 1 shows results of the analysis of cTnT-positive cells by a flow cytometer.
Fig. 2 is a Volcano Plot of a high-producing clone group versus a low-producing clone group.
Fig. 3 is a graph in which the expression amount of NANOG is compared between a high-producing clone group and a low-producing clone group.
Fig. 4 is a graph in which the expression amount of NODAL is compared between the high-producing clone group and the low-producing clone group. * indicates P-value < 0.01.
Fig. 5 is a graph in which the expression amount of LEFTY1 is compared between the high-producing clone group and the low-producing clone group. * indicates P-value < 0.01.
Fig. 6 is a graph in which the expression amount of LEFTY2 is compared between the high-producing clone group and the low-producing clone group. * indicates P-value < 0.01.
Fig. 7 is a graph in which the expression amount of CER1 is compared between the high-producing clone group and the low-producing clone group. * indicates P-value < 0.01.
Fig. 8 is a graph in which the expression amount of CXCR4 is compared between the high-producing clone group and the low-producing clone group. * indicates P-value < 0.01.
Fig. 9 is a graph showing the expression amount of NODAL before differentiation induction into ectoderm.
Fig. 10 is a graph showing the expression amount of PAX6 after differentiation induction into ectoderm.

### DESCRIPTION OF THE PREFERRED EMBODIMENTS

Hereinafter, embodiments for carrying out the present invention will be described in detail.

The abbreviations in the present specification have the following meanings.
bFGF: basic Fibroblast Growth Factor
BMP4: Bone Morphogenetic Protein 4
CER1: Cerberus 1
cTnT: cardiac Troponin T
CXCR4: C-X-C chemokine Receptor type 4
DMEM: Dulbecco's Modified Eagle Medium
DMEM/F12 (DMEM Ham's F-12): Dulbecco's Modified Eagle's Medium / Nutrition Mixture F-12 Ham
D-PBS: Dulbecco's Phosphate Buffered Saline
EDTA: Ethylenediaminetetraacetic acid
Erk: Extracellular Signal-regulated Kinase
ESG: Embryonal stem cell-specific gene
FGF: Fibroblast growth factor
GAPDH: glyceraldehyde-3-phosphate dehydrogenase
GSK: Glycogen Synthesis Kinase
K1f: Kruppel-like factor
LGR5: Leucine-rich repeat-connecting G-protein coupled receptor 5
LIF: Leukemia inhibitory factor
Oct: octamer-binding transcription factor
PAX6: paired box 6
PCR: polymerase chain reaction
ROCK: Rho-associated coiled-coil forming kinase
RT-PCR: Reverse Transcription polymerase chain reaction
SCF: Stem cell factor
Sox: sex determining region Y (SRY)-box
Stat3: Signal Transducer and Activator of Transition 3)
VEGF: Vascular Endothelial Growth Factor

[1] Producing method for a pluripotent stem cell, and a pluripotent stem cell A producing method for a pluripotent stem cell capable of differentiating into a specific cell includes the following steps:
   (i) a step of measuring an expression level of a gene, which is expressed by a primitive endoderm cell, in a pluripotent stem cell as a sample; and
   (ii) a step of comparing the measured expression level of the gene, which is expressed by the primitive endoderm cell, with an expression level of the gene in a pluripotent stem cell known to have high differentiation potency into the specific cell, and selecting a pluripotent stem cell in which the measured expression level of the gene is comparable or low, and acquiring a pluripotent stem cell capable of differentiating into the specific cell.

A pluripotent stem cell capable of differentiating into a specific cell can be selected by using the expression amount of at least one gene expressed by the primitive endoderm cell as an indicator. In a case where a cell that is not capable of differentiating into a specific cell or a cell that is weakly capable of differentiating into a specific cell is removed, the production cost can be reduced, and the production can be stabilized.

The "pluripotent stem cell" refers to a cell having both the ability (the differentiation pluripotency) to differentiate into all cells that constitute a living body and the ability (the self-replication ability) to generate daughter cells having the same differentiation potency as the mother pluripotent stem cell through cell division. The differentiation pluripotency can be evaluated by transplanting an evaluation target cell into a nude mouse and testing for the presence or absence of formation of teratoma that includes cells of the respective three germ layers (ectoderm, mesoderm, and endoderm). The self-replication ability can be evaluated from the fact that, in a case of being subcultured, cells are proliferated and the characteristics of the proliferated cells are not changed by the subculture.

For reference only. Examples of the pluripotent stem cell include an embryonic stem cell (an ES cell), an embryonic germ cell (an EG cell), and an induced pluripotent stem cell (an iPS cell); however, examples thereof are not limited thereto as long as a cell has both differentiation pluripotency and self-replication ability. It is preferable to use an ES cell or an iPS cell, and it is more preferable to use an iPS cell. The pluripotent stem cell is preferably a cell derived from mammals (for example, primates such as a human and a chimpanzee, rodents such as a mouse and a rat), and more preferably a human derived cell. In the most preferred aspect of the present invention, a human iPS cell is used as the pluripotent stem cell.

For reference only. The ES cell can be established, for example, by culturing an early embryo before implantation, an inner cell mass constituting the early embryo, a single blastomere, or the like (Manipulating the Mouse Embryo A Laboratory Manual, Second Edition, Cold Spring Harbor Laboratory Press (1994); Thomson, J. A. et. al., Science, 282, 1145-1147 (1998)). As the early embryo, an early embryo produced by nuclear transplantation of a nucleus of a somatic cell may be used (Wilmut et al. (Nature, 385, 810 (1997)), Cibelli et al. (Science, 280, 1256 (1998)), Iriya et al (Protein Nucleic Acid Enzyme, 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000)), Tachibana et al. (Human Embryonic Stem Cells Derived by Somatic Cell Nuclear Transfer, Cell (2013) in press)). As the early embryo, a parthenogenetic embryo may be used (Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008)). In addition to the above-mentioned papers, the production of the ES cell is described in Strelchenko N., et al. Reprod Biomed Online. 9: 623-629, 2004; KlimanskayaI., et al. Nature 444: 481-485, 2006; Chung Y., et al. Cell Stem Cell 2: 113-117, 2008; Zhang X., et al. Stem Cells 24:2669-2676, 2006; Wassarman P. M., et al. Methods in Enzymology, Vol. 365, 2003, or the like. It is noted that a fused ES cell obtained by cell fusion of an ES cell with a somatic cell is also included in the embryonic stem cell

For reference only. Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO, and the like.

For reference only. An EG cell can be established by culturing a primitive germ cell in the presence of LIF, bFGF, and SCF (Matsui et al., Cell, 70, 841-847 (1992), Shamblott et al., Proc. Natl. Acad. Sci. USA, 95 (23), 13726-13731 (1998), Turnpenny et al., Stem Cells, 21 (5), 598-609, (2003)).

The "induced pluripotent stem cell (the iPS cell)" is a cell that has pluripotency (multiple differentiation potency) and proliferation ability and that is produced by reprogramming a somatic cell by introducing reprogramming factors or the like. The induced pluripotent stem cell exhibit properties similar to the ES cell. The somatic cell that is used for producing an iPS cell is not particularly limited and may be a differentiated somatic cell or an undifferentiated stem cell. In addition, the origin thereof is not particularly limited; however, it is preferable to use a somatic cell of mammals (for example, primates such as a human and a chimpanzee, rodents such as a mouse and a rat), it is more preferable to use a human somatic cell. The iPS cell can be produced by various methods reported so far. In addition, it is naturally expected that an iPS cell producing method to be developed in the future will be applied.

The most basic producing method for an iPS cell is a method in which four transcription factors, Oct3/4, Sox2, Klf4, and c-Myc are introduced into a cell using a virus (Takahashi K, Yamanaka S: Cell 126 (4), 663-676, 2006; Takahashi, K, et al.: Cell 131 (5), 861-72, 2007). It has been reported that human iPS cells have been established by introducing four factors, Oct4, Sox2, Lin28, and Nanog (Yu J, et al.: Science 318 (5858), 1917-1920, 2007). It has also been reported that iPS cells have been established by introducing three factors excluding c-Myc (Nakagawa M, et al.: Nat. Biotechnol. 26 (1), 101-106, 2008), two factors of Oct3/4 and Klf4 (Kim J B, et al.: Nature 454 (7204), 646-650, 2008), or Oct3/4 alone (Kim J B, et al.: Cell 136 (3), 411-419, 2009). In addition, a method for introducing a protein, which is an expression product of a gene, into cells (Zhou H, Wu S, Joo J Y, et al.: Cell Stem Cell 4, 381-384, 2009; Kim D, Kim C H, Moon J I, et al.: Cell Stem Cell 4, 472-476, 2009) has also been reported. On the other hand, it has also been reported that, by using BIX-01294 which is an inhibitor of histone methyltransferase G9a, valproic acid (VPA) which is a histone deacetylase inhibitor, or Bay K8644, the production efficiency has been improved and the factors to be introduced have been reduced (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e253, 2008). Studies on gene transfer methods have also been carried out, and technologies for gene transfer have been developed using, in addition to a retrovirus, a lentivirus (Yu J, et al.: Science 318 (5858), 1917-1920, 2007), an adenovirus (Stadtfeld M, et al.: Science 322 (5903), 945-949, 2008), a plasmid (Okita K, et al.: Science 322 (5903), 949-953, 2008), a transposon vector (Woltjen K, Michael I P, Mohseni P, et al.: Nature 458, 766-770, 2009; Kaji K, Norrby K, Paca A, et al.: Nature 458, et al. 771-775, 2009; Yusa K, Rad R, Takeda J, et al.: Nat Methods 6, 363-369, 2009) or an episomal vector (Yu J, Hu K, Smuga-Otto K, Tian S, et al.: Science 324, 797-801, 2009).

A cell transformed to the iPS cell, that is, a cell that has undergone initialization (reprogramming) can be selected using, as an indicator, the expression of pluripotent stem cell markers (undifferentiated markers) such as Nanog, Oct4, Fgf-4, Esg-1, and Cript, or the like. The selected cell is collected as the iPS cell.

The iPS cells can be provided from, for example, National University Corporation Kyoto University, or Independent Administrative Institution RIKEN BioResource Center.

In the pluripotent stem cells, two types of cells of different states are known; a naive type pluripotent stem cell and a prime type pluripotent stem cell. The naive type pluripotent stem cell and the prime type pluripotent stem cell can be distinguished according to molecular characteristics and cellular characteristics.

Typically, the naive type pluripotent stem cell is characterized by the fact that it expresses high levels of pluripotency factors Oct4, Nanog, Sox2, Klf2, and Klf4, undergoes self-renewal in response to any one of Lif/Stat3 or 2i (ERKi/GSKi), differentiates in response to Fgf/Erk, and exhibits an X-chromosome state of XaXa. Typically, the prime type pluripotent stem cell is characterized by the fact that it expresses high levels of pluripotency factors Oct4, Sox2, and Nanog, does not respond to Lif/Stat3, undergoes self-renewal in response to Fgf/Erk, and exhibits an X chromosome activation state of XaXi (Nichols et al., (2009) Cell Stem Cell 4 (6): 487-492). Here, Xa indicates an active X chromosome, and Xi indicates an inactive X chromosome.

In the present specification, the specific cell is any differentiated cell derived from ectoderm, endoderm, or mesoderm. The differentiated cell derived from the ectoderm is not particularly limited; however, examples thereof include a nervous system cell (a nerve cell, a glial cell, or the like), a sensory organ (crystalline lens, retina, inner ear, or the like) cell, a cutaneous epidermal cell, and a hair follicle.

The differentiated cell derived from the endoderm is not particularly limited; however, examples thereof include a digestive system cell (a liver cell, a bile duct cell, a pancreatic endocrine cell, an acinar cell, a duct cell, an absorptive cell, a goblet cell, a Paneth cell, an intestinal endocrine cell, an intestinal epithelial cell-like cell, or the like) and a cell of a tissue such as lung or thyroid.

The differentiated cell derived from the mesoderm is not particularly limited; however, examples thereof include a hematopoietic cell and a lymphoid cell (a hematopoietic stem cell, an erythrocyte, a platelet, a macrophage, a granulocyte, a helper T cell, a killer T cell, a B lymphocyte, or the like), a vascular system cell (a vascular endothelial cell or the like) a myocardial cell (for example, an atrial cardiac muscle cell, or a ventricular cardiac muscle cell), an osteoblast, a bone cell, a cartilage cell, a tendon cell, an adipocyte, a skeletal muscle cell, and a smooth muscle cell.

The step (i) in the producing method for a pluripotent stem cell according to the embodiment of the present invention is a step of measuring an expression level of a gene, which is expressed by a primitive endoderm cell, in a pluripotent stem cell as a sample.

In the description of the "pluripotent stem cell as a sample", "as a sample" means that the sample is capable of being a material for producing a pluripotent stem cell capable of differentiating into a specific cell, and from the "pluripotent stem cell as a sample", a pluripotent stem cell capable of differentiating into a specific cell is acquired by the step (ii) described later.

The primitive endoderm cell is a cell that differentiates in the surface layer of the inner cell mass of the blastocyst, which faces the blastocyst cavity. The gene expressed by the primitive endoderm cell may be a gene specifically expressed bv the primitive endoderm cell. The gene specifically expressed by the primitive endoderm cell means a gene of which expression is detected in the primitive endoderm of a blastocyst, but of which expression is not detected in the upper layer of the blastoderm. In the present invention, as the gene expressed by the primitive endoderm cell, at least one gene selected from the group consisting of CER1, LEFTY1, LEFTY2, NODAL, and CXCR4 is used.

In WO2014/200030A, a target iPS cell of which expression levels of genes such as GATA4 and GATA6 are comparable to or higher than those in an iPS cell or the like is screened as an iPS cell that exhibits a high differentiation efficiency into a hematopoietic stem cell and/or a hematopoietic progenitor cell, where the iPS cell or the like is known to exhibit a high differentiation efficiency into a hematopoietic stem cell and/or hematopoietic progenitor cell. On the other hand, as described below, the producing method for a pluripotent stem cell capable of differentiating into the specific cell according to the embodiment of the present invention is different from the method described in WO2014/200030A in that an expression level of the above gene is compared with an expression level of the gene in the pluripotent stem cell known to have high differentiation potency into the specific cell, and a pluripotent stem cell in which the measured expression level of the gene is comparable or low is selected, whereby a pluripotent stem cell capable of differentiating into the specific cell is acquired.

The expression level of a gene means the expression amount of a gene, and can be usually analyzed by the production amount of a transcript corresponding to the gene, or the production amount, the activity, and the like of a translation product thereof. The measurement of the expression level can be carried out by measuring an mRNA which is a transcript of a gene or a protein which is a translation product of a gene; however, it is preferably carried out by measuring an mRNA or a cDNA which is a reverse transcript thereof.

Table 1 shows the NCBI accession number of each gene.

**[Table 1]**

| Gene name | NCBI accession Number | |
|---|---|---|
| | Human | Mouse |
| CER1 | NM_005454.2 | NM_009887.2 |
| LEFTY1 | NM_020997.4 | NM_010094.4 |
| LEFTY2 | NM_003240.5 | NM_177099.4 |
| NODAL | NM_018055.4 | NM_013611.5 |
| CXCR4 | NM_001008540.2 | NM_009911.3 |

Based on the above NCBI accession number, the sequence information of each gene can be acquired, and the expression level of the gene can be measured.

The method for measuring an expression level of a gene expressed by the primitive endoderm cell is not particularly limited; however, the measurement can be carried out by, for example, quantitative RT-PCR. The RT-PCR is a method of synthesizing cDNA using the measurement target mRNA as a template and amplifying it by PCR using this cDNA as a template. Examples of the quantitative RT-PCR include a method (a real-time PCR) in which PCR is carried out using a primer to which a quencher fluorescent dye and a reporter fluorescent dye are bound to quantify the amount of an amplification product in each cycle, and the amount of a template DNA in a sample is measured from the number of cycles at which the detected fluorescence intensity increases sharply. The quantitative RT-PCR technique is well known in the technical field of the present invention and can also be carried out using a commercially available kit. According to the quantitative RT-PCR, the expression amount or the number of copies of a gene can be measured as a relative value with respect to the expression amount or the number of copies of a control housekeeping gene (for example, a GAPDH gene). The mRNA of a gene can also be measured by subjecting an amplification product obtained by amplifying the mRNA by the ordinary RT-PCR or the like to gel electrophoresis, staining the gel, and then measuring the band intensity. Alternatively, a DNA chip can be used to detect or quantify the mRNA or cDNA of a gene. The expression level of a gene expressed by the primitive endoderm cell can also be measured using a next-generation sequencer. A measurement target cell can be obtained by the partial extraction of cells in the culture step.

As the numerical value indicating the expression level, for example, a cycle threshold (Ct) value can be used in a case where the expression amount is measured by the real-time PCR. The Ct value is the number of cycles at which a PCR amplification product reaches a certain amount. In a case where the number of cycles of amplification is plotted on the horizontal axis and the amount of a PCR product is plotted on the vertical axis to create an amplification curve, and a threshold is set for the value of the PCR product, the number of cycles at the point where the threshold and the amplification curve intersect is the Ct value. In a case of measuring the expression amount using a fluorescently labeled probe, fluorescence intensity can also be used.

In the producing method for a pluripotent stem cell according to the embodiment of the present invention, first, a pluripotent stem cell can be subjected to expansion culture. The "pluripotent stem cell as a sample" is preferably subjected to the expansion culture in a suitable medium on a plate coated with a feeder cell or a plate coated with a scaffold such as Matrigel (registered trade name). The feeder cell is not particularly limited; however, examples thereof include a mouse embryonic fibroblast (an MEF cell) and a mouse embryonic fibroblast (an STO cell). In the expansion culture, it is preferable that the undifferentiative property of a cell is maintained.

As the medium for the expansion culture, a commercially available medium such as mTeSR (registered trade name) 1 (STEMCELL Technologies Inc.) or StemFlex (registered trade name) can be used. Alternatively or additionally, for example, DMEM, a mixed medium of DMEM and F12 (DMEM/F12 = 1:1), and Knockout^{™} D-MEM (Thermo Fisher Scientific, Inc.) can be mentioned as the basal medium, and a medium prepared by adding, to any one of the above basal media, any combination of components such as alternative serum (KSR; Knockout^{™} Serum Replacement (Thermo Fisher Scientific, Inc.)), fetal bovine serum (FBS), non-essential amino acid (NEAA), L-glutamine, 2-mercaptoethanol, and an antibiotic (for example, streptomycin, penicillin, puromycin, mitomycin, or gentamycin), and bFGF can be mentioned as a medium for the expansion culture.

The culture conditions for the expansion culture are preferably conditions of 37°C, 5% CO₂, and 10% Oz, or the like; however, they are not particularly limited.

By the expansion culture, cells can be cultured until the cell concentration in the culture solution changes from 1 × 10⁴ cells/mL to 1 × 10¹⁰ cells/mL.

Subculture may be carried out during the expansion culture. For example, in a case where cells become confluent or subconfluent, a part of the cells can be collected and transferred to another culture vessel, and the culture can be continued. It is preferable to set a cell density low in order to promote differentiation. For example, cells may be plated at a cell density of about 1 × 10⁴ cells/cm² to 1 × 10⁶ cells/cm².

The pluripotent stem cell as a sample is a cell obtained by clonally culturing and proliferating a pluripotent stem cell by the expansion culture. The clonal culture means a culture for obtaining a population of cells, which is formed through the division and the proliferation of one cell by culture, and the cell population obtained by the clonal culture is called a clonal population or simply a clone.

The expression level of a gene expressed by the primitive endoderm cell can be measured for naive type pluripotent stem cell or prime type pluripotent stem cell; however, it is preferably carried out for the prime type pluripotent stem cell. This is because the prime type pluripotent stem cell is suitable for detecting the expression of a gene, which is expressed by the primitive endoderm cell, in the pluripotent stem cell.

The step (ii) in the producing method for a pluripotent stem cell according to the embodiment of the present invention is a step of comparing an expression level of a gene, where the expression level is measured in the step (i) and the gene is expressed by the primitive endoderm cell, with an expression level of the gene in a pluripotent stem cell known to have high differentiation potency into the specific cell, and selecting a pluripotent stem cell in which the measured expression level of the gene is or low, as defined in the appended claims and acquiring a pluripotent stem cell capable of differentiating into the specific cell. In addition, in the step (ii), the expression level of the gene expressed by the primitive endoderm cell, which has been measured in the step (i), is compared with an expression level of the gene in a pluripotent stem cell known to have high differentiation potency into the specific cell, and a cell having a high expression level is not selected.

The "pluripotent stem cell known to have high differentiation potency into the specific cell" means a pluripotent stem cell that has been revealed that the differentiation potency into the specific cell is relatively high, by the reported publication such as literature or the experiment carried out in advance for evaluating the differentiation potency into the specific cell.

Examples of the "pluripotent stem cell known to have high differentiation potency into the specific cell" include a 01434 strain (McNamara et al., 2018, Cell Systems 7, 359-370) of Fujifilm Cellular Dynamics, Inc.

The selection of the "pluripotent stem cell known to have high differentiation potency into the specific cell" can be carried out, for example, by determining the proportion (the specific cell production rate) of the number of specific cells after the differentiation induction into the specific cell with respect to the number of pluripotent stem cells at the start of the differentiation induction and selecting a cell having a relatively high specific cell production rate. Since the specific cell production rate can be changed depending on the kind of the specific cell and the differentiation inducing method, it is possible to set a value of the specific cell production rate, which is the reference for selecting "the pluripotent stem cell having high differentiation potency into the specific cell" for each kind of specific cell and each kind of the differentiation inducing method. A person skilled in the art can appropriately set the specific cell production rate for dividing cells that "have high differentiation potency to the specific cell" and cells that do not have.

The "pluripotent stem cell known to have high differentiation potency into the specific cell" can be selected, for example, by evaluating the production rate of a differentiated cell or a progenitor cell thereof, which is derived from mesoderm, endoderm, or ectoderm, and preferably a differentiated cell or a progenitor cell thereof, which is derived from mesoderm or ectoderm. More specifically, the differentiation potency of the pluripotent stem cell into the specific cell can be evaluated by inducing differentiation into, for example, the myocardial cell, a blood cell, a skeletal muscle cell, a nerve cell, a hepatic parenchymal cell, a pancreatic β cell, an intestinal epithelial cell, or a progenitor cell thereof. This is because there is an established experimental system for inducing differentiation of these differentiated cells or progenitor cells thereof.

An example of the pluripotent stem cell known to have high differentiation potency into the specific cell is the pluripotent stem cell known to have high differentiation potency into the myocardial cell. Examples of the pluripotent stem cell known to have high differentiation potency into the myocardial cell include a pluripotent stem cell preferably having a myocardial cell production rate of 50% or more, and include a pluripotent stem cell more preferably having a myocardial cell production rate of 60% or more, 70% or more, 80% or more, 90% or more, 100% or more, 110% or more, 120% or more, or 130% or more. In addition, a pluripotent stem cell having a myocardial cell production rate of 140% or more, 150% or more, 160% or more, 170% or more, 180% or more, 190% or more, or 200% or more may be used.

The myocardial cell production rate (%) means (the total number of myocardial cells after differentiation induction / the number of pluripotent stem cells at the start of differentiation induction) × 100.

As will be described later, the differentiation induction into the myocardial cell can be carried out as follows; cells are cultured in mTeSR (registered trade name) 1, to which a ROCK inhibitor and bFGF are added, on the 1st day, cultured in a × 0.5 mTeSR (registered trade name) 1 and × 0.5 DMEM low-glucose medium containing a ROCK inhibitor, bFGF, Activin A, and fetal bovine serum, on the 2nd day, cultured in a DMEM low-glucose medium containing bFGF, Activin A, BMP4, and fetal bovine serum on the 3rd day to 8th day, cultured in the same medium containing a Wnt inhibitor and fetal bovine serum on the 9th day, and cultured in the same medium without a Wnt inhibitor on the 10th day to 14th day.

The myocardial cell can be identified by detecting a cell expressing cTnT which is a myocardial cell marker, for example, using an anti-cTnT antibody labeled with a fluorescent dye. The number of myocardial cells can be calculated by measuring the proportion (the cTnT-positive cell rate) of cells expressing cTnT by flow cytometry method using an anti-cTnT antibody or the like through the labeling with a fluorescent dye. The cTnT-positive cell rate can be measured by the flow cytometry method according to the method described in Example 2.

Examples of the pluripotent stem cell known to have high differentiation potency into the myocardial cell include a pluripotent stem cell preferably having a cTnT-positive cell rate after differentiation induction of 25% or more, and include a pluripotent stem cell more preferably having a cTnT-positive cell rate after differentiation induction 30% or more, 35% or more, 40% or more, 45% or more, 50% or more, 55% or more, or 60% or more.

In the present invention, an expression level of a gene, which is expressed by the primitive endoderm cell, is compared with an expression level of the gene in the pluripotent stem cell known to have high differentiation potency into the specific cell, and a pluripotent stem cell in which the expression level is or low as defined in the appended claims is selected.

"Comparable" means that an expression level of a gene expressed by the primitive endoderm primitive endoderm cell is 1 time or more and less than 2 times, and preferably 1.9 times or less, 1.8 times or less, 1.7 times or less, 1.6 times or less, 1.5 times or less, 1.4 times or less, 1.3 times or less, 1.2 times or less, or 1.1 times or less, relative to the expression level of the gene in the pluripotent stem cell known to have high differentiation potency into the specific cell.

"Low" means that an expression level of a gene expressed by the primitive endoderm cell is less than 1 time and is preferably 0.9 times or less, 0.8 times or less, 0.7 times or less, or 0.6 times or less, relative to the expression level in the pluripotent stem cell known to have high differentiation potency into the specific cell.

In a case where the gene expressed by the primitive endoderm cell is a NODAL gene, an expression level of the NODAL gene in the pluripotent stem cell to be selected is preferably 0.9 times or less, 0.8 times or less, 0.7 times or less, or 0.6 times or less, relative to the expression level of the NODAL gene in the pluripotent stem cell known to have high differentiation potency into the specific cell.

In a case where the gene expressed by the primitive endoderm cell is a LEFTY1 gene, an expression level of the LEFTY1 gene in the pluripotent stem cell to be selected is preferably 0.9 times or less, 0.8 times or less, 0.7 times or less, or 0.6 times or less, relative to the expression level of the LEFTY1 gene in the pluripotent stem cell known to have high differentiation potency into the specific cell.

In a case where the gene expressed by the primitive endoderm cell is a LEFTY2 gene, the expression level of the LEFTY2 gene in the pluripotent stem cell to be selected is preferably 0.9 times or less, 0.8 times or less, 0.7 times or less, or 0.6 times or less, relative to the expression level of the LEFTY2 gene in the pluripotent stem cell known to have high differentiation potency into the specific cell.

In a case where the gene expressed by the primitive endoderm cell is a CER1 gene, an expression level of the CER1 gene in the pluripotent stem cell to be selected is preferably 0.9 times or less, 0.8 times or less, 0.7 times or less, or 0.6 times or less, relative to the expression level of the CER1 gene in the pluripotent stem cell known to have high differentiation potency into the specific cell.

In a case where the gene expressed by the primitive endoderm cell is a CXCR4 gene, an expression level of the CXCR4 gene in the pluripotent stem cell to be selected is preferably 0.9 times or less, 0.8 times or less, 0.7 times or less, or 0.6 times or less, relative to the expression level of the CXCR4 gene in the pluripotent stem cell known to have high differentiation potency into the specific cell.

A person skilled in the art can appropriately set a reference value for selecting a pluripotent stem cell having differentiation potency into the specific cell based on the expression level of each gene in the pluripotent stem cell known to have high differentiation potency into the specific cell. For example, an average value of expression levels of a gene, which is expressed by the primitive endoderm cell, in two or more clones selected as the "pluripotent stem cell known to have high differentiation potency into the specific cell" is used as a reference value, the expression level of the gene is compared with this reference value, and a pluripotent stem cell in which the expression level is comparable or low can be selected as the pluripotent stem cell having differentiation potency into the specific cell. The reference value can be set for each gene.

In the selection of the pluripotent stem cell having differentiation potency into the specific cell, the expression levels of two or more genes expressed by the primitive endoderm primitive endoderm cell as a sample are measured, scored by applying discriminant analysis, and then it is possible to determine whether or not the primitive endoderm cell as a sample cell is the pluripotent stem cell having differentiation potency into the specific cell. Examples of the discriminant analysis include Fisher's linear discrimination, Mahalanobis' distance-based discrimination, Euclidean distance-based discrimination, multigroup discrimination, variable selection discrimination, and canonical discrimination. These discriminant analyzes can be carried out using various statistical analysis software.

A pluripotent stem cell capable of differentiating into the specific cell, which is obtained by the method described above. In a case where the pluripotent stem cell capable of differentiating into the specific cell, which is obtained by the above production method, is induced to differentiate, it is possible to efficiently produce a differentiated cell.

A method for screening a pluripotent stem cell capable of differentiating into a specific cell, includes the following steps:
(i) a step of measuring an expression level of a gene, which is expressed by a primitive endoderm cell, in a pluripotent stem cell as a sample; and
(ii) a step of comparing the measured expression level of the gene, which is expressed by the primitive endoderm cell, with an expression level of the gene in a pluripotent stem cell known to have high differentiation potency into the specific cell, and selecting a pluripotent stem cell in which the measured expression level of the gene is comparable or low, and acquiring a pluripotent stem cell capable of differentiating into the specific cell.

A method for quality evaluation of a pluripotent stem cell, includes
(a) a step of measuring an expression level of a gene, which is expressed by a primitive endoderm cell, in a pluripotent stem cell as a sample;
(b) a step of comparing the measured expression level of the gene, which is expressed by the primitive endoderm cell, with an expression level of the gene in a pluripotent stem cell known to have high differentiation potency into the specific cell; and
(c) a step of determining that a pluripotent stem cell in which the measured expression level of the gene is comparable or low is excellent.

The quality evaluation of the present invention means predicting whether or not the pluripotent stem cell as a sample has high differentiation potency into the specific cell, before the differentiation induction. Using the expression level of at least one kind of gene expressed by the primitive endoderm cell as an indicator, it is possible to evaluate that a pluripotent stem cell as a sample, which is predicted to have high differentiation potency into the specific cell, is an excellent strain and that a pluripotent stem cell as a sample, which is predicted not to have high differentiation potency into the specific cell, is a defective strain.

According to the method for quality evaluation according to the embodiment of the present invention, the production cost can be reduced, and the production can be stabilized by excluding the defective strains by the quality evaluation. In addition, in a case where a part of cells are sampled at a plurality of time points in the clonal culture process to monitor the progress of the expression amount of a gene, which is expressed in the primitive endoderm cell, the deterioration of cell quality in the clonal culture process can be detected, which can also be fed back to the production process. In each of the steps of establishing, expanding, and differentiating an iPS cell, the expression amount of the primitive endodermal gene can be measured and monitored as a novel quality reference of the iPS cell.

The step (a) in the present invention can be carried out in the same manner as the step (i) described in [1] Producing method. The step (b) in the present invention can be carried out in the same manner as the step (ii) described in [1] Producing method. In step (b), the expression level of the gene expressed by the primitive endoderm cell, which has been measured in the step (a), is compared with an expression level of the gene in a pluripotent stem cell known to have high differentiation potency into the specific cell, a pluripotent stem cell in which the measured expression level of the gene is comparable or low, can be evaluated as the pluripotent stem cell capable of differentiating into the specific cell. It is preferable that the pluripotent stem cell evaluated to be capable of differentiating into the specific cell is a differentiation induction target. This makes it possible to efficiently produce a differentiated cell product.

### [2] Producing method differentiated cell, and differentiated cell

A producing method for a differentiated cell includes the following steps.

A step of obtaining a pluripotent stem cell capable of differentiating into a specific cell by the method for producing a pluripotent stem cell capable of differentiating into a specific cell, and
a step of inducing differentiation of the pluripotent stem cell capable of differentiating into a specific cell, obtained in the above step.

In a case where the pluripotent stem cell capable of differentiating into the specific cell, which is obtained by the producing method for a pluripotent stem cell capable of differentiating into the specific cell according to the embodiment of the present invention as defined in the appended claims, is induced to differentiate, it is possible to efficiently produce a differentiated cell.

A step of obtaining a pluripotent stem cell capable of differentiating into a specific cell by the method for producing a pluripotent stem cell capable of differentiating into a specific cell can be carried out based on the description of [1] Producing method.

The "inducing differentiation" refers to acting to differentiate along a specific cell lineage. The method of inducing differentiation into a pluripotent stem cell capable of differentiating into a specific cell is not particularly limited. For example, a commercially available StemDiff (registered trade name) Trillineage Differentiation Kit (STEMCELL Technologies Inc.) can be used to induce differentiation into each of the endoderm, mesoderm, and ectoderm.

In the differentiation induction into an endodermal cell, for example, a cell can be induced to differentiate into the endodermal cell by using a STEMdiff (registered trade name) Trilineage Endoderm Medium (STEMCELL Technologies Inc.) according to the procedure manual.

In the differentiation induction into a mesodermal cell, for example, a cell can be induced to differentiate into the mesodermal cell by using a STEMdiff (registered trade name) Trilineage Mesoderm Medium (STEMCELL Technologies Inc.) according to the procedure manual.

The differentiation induction into the ectodermal cell can be carried out, for example, by culturing cells under the conditions described in Example 5 described later. Specifically, a cell can be induced to differentiate into the ectodermal cell by using a STEMdiff (registered trade name) Trilineage Ectoderm Medium (STEMCELL Technologies Inc.) according to the procedure manual.

The confirmation of differentiation induction into the endodermal cell, the mesodermal cell, and the ectodermal cell can be confirmed by the expression of each germ layer-specific gene. The method of measuring the expression of each germ layer-specific gene is not particularly limited; however, the measurement can be carried out by, for example, a quantitative RT-PCR method.

The endoderm-specific gene is not particularly limited; however, examples thereof include SOX17 and FOXA2. The mesoderm-specific gene is not particularly limited; however, examples thereof include T and PDGFRA. The ectoderm-specific gene is not particularly limited; however, examples thereof include PAX6 and MAP2.

The differentiation induction into the myocardial cell can be carried out, for example, by culturing cells under the conditions described in Example 1 described later. Specifically, the differentiation induction into the myocardial cell can be carried out as follows; cells are cultured in mTeSR (registered trade name) 1, to which a ROCK inhibitor and bFGF are added, on the 1st day, cultured in a × 0.5 mTeSR (registered trade name) 1 and × 0.5 DMEM low-glucose medium containing a ROCK inhibitor, bFGF, Activin A, and fetal bovine serum, on the 2nd day, cultured in a DMEM low-glucose medium containing bFGF, Activin A, BMP4, and fetal bovine serum on the 3rd day to 8th day, cultured in the same medium containing a Wnt inhibitor and fetal bovine serum on the 9th day, and cultured in the same medium without a Wnt inhibitor on the 10th day to 14th day. As the ROCK inhibitor, for example, H1152, Y27634, and the like can be used. As the Wnt inhibitor, for example, XAV939 can be used. Alternatively, it is also possible to induce differentiation into the myocardial cell using a PSC Cardiomyocyte Difference Kit (Thermo Fisher Scientific, Inc.) according to the procedure manual. The differentiation induction into the myocardial cell can be confirmed by measuring the expression of the myocardial cell marker cTnT by flow cytometry.

In addition, the differentiation induction into the blood cell can be carried out by culturing cells under the conditions described in WO2019/151386A. Specifically, the differentiation induction into the blood cell can be carried out as follows; cells are cultured in a medium containing BMP4 and Y27634 (a ROCK inhibitor) on the 1st day, bFGF and BMP4 are added on the second day, after confirming the formation of spheroid-like colonies on the 3rd day, the cells are cultured in a medium containing SB431542 (a TGF-β receptor inhibitor), CHIR99021 (a GSK3 inhibitor), bFGF, and BMP4 (on the 3rd day and 4th day), cultured in a medium containing VEGF and bFGF on the 5th day and 6th day, and cultured in a medium containing VEGF, bFGF, IL-6, IGF-1, IL-11, and SCF on the 7th day to 10th day. The differentiation induction into the blood cell can be confirmed by analyzing the expression of CD34 and KDR, which are markers of the blood cell, with a flow cytometer.

The differentiation induction into the neural stem cell can be carried out, for example, by culturing cells under the conditions described in WO2019/151386A. Specifically, cells can be induced to differentiate into the neural stem cell by using a PSC Natural Induction Medium (Thermo Fisher Scientific, Inc.) according to the procedure manual. The differentiation induction into the neural stem cell can be confirmed, for example, by immunostaining the SOX1 protein, which is a marker of the neural stem cell.

The differentiation induction into the intestinal stem cell-like cell can be carried out, for example, by culturing cells under the conditions described in WO2019/156200A. Specifically, cells can be cultured in DMEM/F12 containing FGF2 or a GSK-3β inhibitor to induce differentiation into the intestinal stem cell-like cell. The differentiation induction into the intestinal stem cell-like cell can be confirmed, for example, by immunostaining the LGR5 protein, which is a marker of the intestinal stem cell.

The differentiation induction into the intestinal epithelial cell-like cell can be carried out, for example, by culturing cells under the conditions described in WO2019/156200A. Specifically, cells can be cultured in Advanced DMEM/F-12 containing EGF and forskolin to induce differentiation into the intestinal epithelial cell-like cell. The differentiation induction into the intestinal epithelial cell-like cell can be determined or evaluated using, for example, the expression of an intestinal epithelial cell marker, the incorporation of a peptide, or the induction of expression of a drug metabolizing enzyme via a vitamin D receptor as an indicator.

In a case of being cultured under differentiation conditions to the mesodermal cell other than the conditions described above, according to the producing method for a differentiated cell according to the embodiment of the present invention as defined in the appended claims, the pluripotent stem cell capable of differentiating into the specific cell can be allowed to differentiate into cells derived from mesoderm. The differentiated cell derived from the mesoderm is not particularly limited; however, examples thereof include a hematopoietic cell and a lymphoid cell (a hematopoietic stem cell, an erythrocyte, a platelet, a macrophage, a granulocyte, a helper T cell, a killer T cell, a B lymphocyte, or the like), a vascular system cell (a vascular endothelial cell or the like) a myocardial cell (for example, an atrial cardiac muscle cell, or a ventricular cardiac muscle cell), an osteoblast, a bone cell, a cartilage cell, a tendon cell, an adipocyte, a skeletal muscle cell, and a smooth muscle cell.

In a case of being cultured under differentiation conditions to the ectodermal cell other than the conditions described above, according to the producing method for a differentiated cell according to the embodiment of the present invention as defined in the appended claims, the pluripotent stem cell capable of differentiating into the specific cell can be allowed to differentiate into cells derived from ectoderm. The differentiated cell derived from the ectoderm is not particularly limited; however, examples thereof include a nervous system cell (a nerve cell, a glial cell, or the like), a sensory organ (crystalline lens, retina, inner ear, or the like) cell, a cutaneous epidermal cell, and a hair follicle.

In a case of being cultured under differentiation conditions to the endodermal cell, according to the producing method for a differentiated cell according to the embodiment of the present invention, as defined in the appended claims the pluripotent stem cell capable of differentiating into the specific cell can be allowed to differentiate into cells derived from endoderm. The differentiated cell derived from the endoderm is not particularly limited; however, examples thereof include digestive system cell (a liver cell, a bile duct cell, a pancreatic endocrine cell, an acinar cell, a duct cell, an absorptive cell, a goblet cell, a Paneth cell, an intestinal endocrine cell, an intestinal epithelial cell, or the like) and a cell of a tissue such as lung or thyroid.

A differentiated cell is obtained by the above producing method for a differentiated cell. The cell induced to differentiate according to the producing method for a differentiated cell according to the embodiment of the present invention can be used for screening pharmaceutical drug candidate compound for the treatment of various diseases. For example, in a case where a pharmaceutical drug candidate compound is added, alone or in combination with other drugs, to cells which have been induced to differentiate, it is possible to carry out evaluation by detecting change in the morphology or function of the cell, the increase or decrease in various factors, the gene expression profiling, or the like.

The cell induced to differentiate according to the producing method for a differentiated cell according to the embodiment of the present invention can be used to prepare a tissue that can be used in the field of regenerative medicine. A person skilled in the art shall be familiar with the method of transplanting the prepared tissue to a patient.

The present invention as defined in the appended claims will be further specifically described with reference to Examples; however, the present invention is not limited by Examples.

### Examples

### <Example 1>

### Differentiation induction into myocardial cell

IPS cell clones A to N were prepared from peripheral blood mononuclear cells having different donor origins according to the method described in JP5984217B. mTeSR (registered trade name) 1 (STEMCELL Technologies Inc.) and Matrigel (registered trade name) (Corning Inc.) were used for culturing iPS cells, and the cells were treated with a 0.5 mmol/L EDTA solution (Thermo Fisher Scientific, Inc.) for 7 minutes for collection and then subcultured. Under the above conditions, the iPS cells were subjected to the expansion culture up to two T225 flasks. The iPS cells obtained as described above are of prime type.

The iPS cells were proliferated under the above conditions until the confluency reached about 80%, the cells were subsequently detached to be single cells with TrypLE (registered trade name) and collected, and the cell concentration was adjusted to 3.0 × 10⁶ cells/mL in mTeSR (registered trade name) 1 to which 1 µmol/L H1152 (FUJIFILM Wako Pure Chemical Corporation), 25 µg/mL gentamycin (Thermo Fisher Scientific, Inc.), and 100 ng/mL bFGF (FUJIFILM Wako Pure Chemical Corporation) were added in terms of final concentration. 15 mL of the suspension of the above cells was added to a 30 mL single-use bioreactor (ABLE Corporation) and subjected to spinner culture at a rotation speed of 40 rpm. After 2 to 4 hours from the start of the culture, the cells were diluted in the same medium so that the final liquid volume was 30 mL and the number of cells was 4.5 × 10⁷ cells, and then the spinner culture was continued as it was.

1 day after the start of the spinner culture, the medium was replaced with a medium consisting of, in terms of final concentration, 1 µmol/L H1152, 25 µg/mL gentamycin, 100 ng/mL bFGF, 24 ng/mL Activin A (R&D Systems Inc.), 5% fetal bovine serum (GE Healthcare), × 0.5 mTeSR (registered trade name) 1, and × 0.5 DMEM low-glucose (Thermo Fisher Scientific, Inc.), and then the spinner culture was continued.

2 days after the start of the spinner culture, a part of the culture solution was sampled to measure the number of cells, the cell concentration was adjusted to 1.0 × 10⁶ cells/mL in a medium consisting of, in terms of final concentration, 25 µg/mL gentamycin, 100 ng/mL bFGF, 24 ng/mL Activin A, 40 ng/mL BMP4 (R&D Systems Inc.), 10% fetal bovine serum, and DMEM low-glucose, and then the spinner culture was continued. From 3 days to 7 days after the start of the spinner culture, the medium was changed daily with the same medium, and then the spinner culture was continued.

8 days after the start of the spinner culture, a part of the culture solution was sampled to measure the number of cells, the cell concentration was adjusted to 1.0 × 10⁶ cells/mL in a medium consisting of, in terms of final concentration, 25 µg/mL gentamycin, 16.25 µg/mL XAV939 (Sigma-Aldrich Co., LLC), 10% fetal bovine serum, and DMEM low-glucose, and then the spinner culture was continued. From 9 days to 13 days after the start of the spinner culture, the medium was changed every two days with the same medium excluding XAV939, and then the spinner culture was continued.

14 days after the start of the spinner culture, it was confirmed that parts of cell aggregates derived from clones A, B, D, E, F, G, I, J, K, L, M, and N, respectively, were autonomously beating. On the other hand, no beating was observed in cell aggregates derived from clones C and H. A part of the culture solution was sampled to measure the number of cells. The number of cells finally obtained is shown in Table 2.

**[Table 2]**

| Clone name | Total number of cells after differentiation induction into myocardial cell (× 10⁶ cells) |
|---|---|
| A | 143.5 |
| B | 534.7 |
| C | 4.9 |
| D | 24.8 |
| E | 573.5 |
| F | 212.9 |
| G | 334.2 |
| H | 8.4 |
| I | 315.4 |
| J | 173.1 |
| K | 94.6 |
| L | 555.2 |
| M | 285.6 |
| N | 46.6 |

### <Example 2>

### Analysis of iPS cell-derived myocardial cell aggregate by flow cytometer

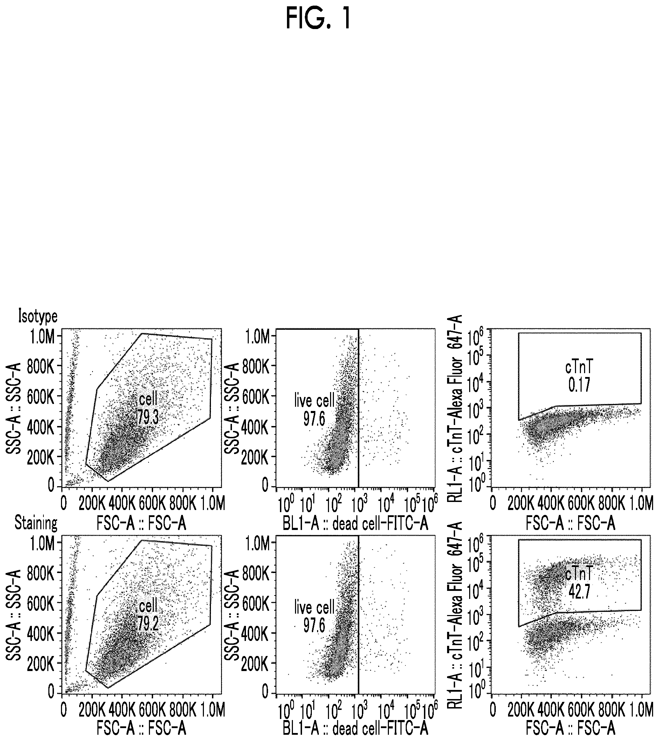

The cell aggregate 14 days after the start of the spinner culture was separated into single cells by TrypLE (registered trade name) Select, and then dead cells were stained with a Live/Dead (registered trade name) Fixable Green Dead Cell Stain Kit (Thermo Fisher Scientific, Inc.). After washing with D-PBS (Thermo Fisher Scientific, Inc.), cells were treated with formaldehyde (Sigma-Aldrich Co., LLC) at a final concentration of 4% to fix the cells. An anti-cTnT antibody (Abcam plc, ab8295) was diluted to 1/250 in D-PBS containing 0.1% Saponin (Merck Millipore) and 2% fetal bovine serum in terms of final concentration, added to 0.5 × 10⁶ cells of the obtained fixed cell, and treated at room temperature for 1 hour. At the same time, as the isotype control, a sample to which IgG1 isotype Control Murine Myeloma (Sigma-Aldrich Co., LLC, M5284) diluted to 1/25 had been added was arranged in parallel. Subsequently, an Alexa Fluor (registered trade name) 647-labeled Goat anti-mouse IgG1 antibody (Thermo Fisher Scientific, Inc., A21240) was diluted to 1/500, added to the cells, and treated at room temperature for 30 minutes. The obtained labeled cells were analyzed using a flow cytometer (Thermo Fisher Scientific, Inc., Attune Nxt). After gating with forward light scattering and lateral light scattering, and then gating live cells, the cTnT-positive cell rate was calculated from the comparison with the isotype control sample. Examples of the flow cytometry result and the gating of the obtained clone A are shown in Fig. 1. Table 3 shows the cTnT-positive cell rate of each clone after the differentiation induction into the myocardial cell, the total number of myocardial cells (the number of cTnT-positive cells) calculated from the total number of cells and the cTnT-positive cell rate after differentiation induction, and the myocardial cell production rate (%) ((the total number of myocardial cells after differentiation induction / the number of pluripotent stem cells at the start of differentiation induction) × 100) based on the number of pluripotent stem cells at the start of differentiation induction.

**[Table 3]**

| Clone name | cTnT-positive cell rate (%) | Total number of myocardial cells (× 10⁶ cells) | Myocardial cell production rate (%) |
|---|---|---|---|
| A | 42.7 | 61.3 | 136.2 |
| B | 14.4 | 77.0 | 171.1 |
| C | 0.1 | 0.0 | 0.0 |
| D | 22.8 | 5.6 | 12.5 |
| E | 25.5 | 146.2 | 325.0 |
| F | 16.8 | 35.8 | 79.5 |
| G | 62.2 | 207.9 | 461.9 |
| H | 1.1 | 0.1 | 0.2 |
| I | 44.9 | 141.6 | 314.7 |
| J | 41.6 | 72.0 | 160.0 |
| K | 40.1 | 37.9 | 84.3 |
| L | 11.6 | 64.4 | 143.1 |
| M | 41.8 | 119.4 | 265.3 |
| N | 16.9 | 7.9 | 17.5 |

### <Example 3>

### Gene expression analysis of iPS cell

At the time of the expansion culture of the iPS cell of Example 1, a part of cells were sampled, and RNA was extracted using an RNeasy Plus Mini Kit (QIAGEN). The RNA was analyzed using a microarray analysis Clariom S, Human Assay (Affymetrix, Inc.) to obtain a gene expression amount matrix for each iPS cell clone. Data analysis was carried out according to Transcriptome Analysis Console (Thermo Fisher Scientific, Inc.).

### <Example 4>

### Correlation analysis between gene expression of iPS cell and myocardial cell production rate

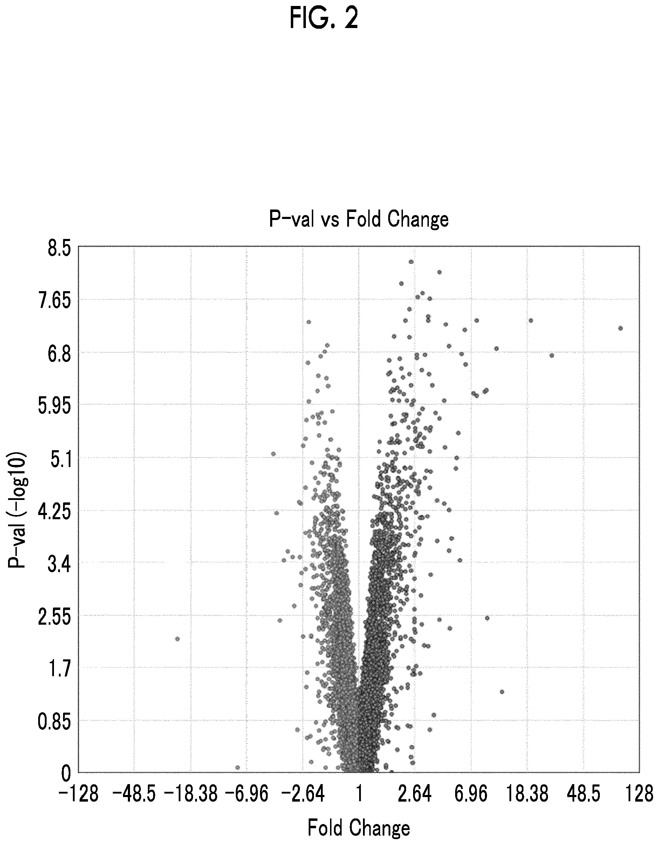

In order to compare the production rate of the myocardial cell derived from each iPS cell clone, obtained according to Example 2, and the gene expression amount obtained according to Example 3, clones A, B, E, F, G, I, J, K, L, and M were defined as the myocardial cell high-producing clone group, clones C, D, H, and N were defined as the myocardial cell low-producing clone group, and a Volcano Plot (a comparison between two groups by combining t-test and fold analysis) was created. The results are shown in Fig. 2. In Fig. 2, the vertical axis indicates the P-value (-log 10), which is the significance level of the t-test, and the horizontal axis indicates the Fold Change, which is the ratio of the average values.

In addition, genes having an absolute value of expression amount difference of 2 times or more and a significant difference of expression (the P-value) of less than 0.01 between the myocardial cell high-producing clone group and the myocardial cell low-producing clone group were extracted and listed (the list is not attached). From the obtained gene list, it was found that the iPS cell of the low-producing clone group tends to have significantly higher expression of genes that are said to be expressed in the primitive endoderm, such as NODAL, CER1, and CXCR4. In addition, it was also found that when the expression amounts of LEFTY1 and LEFTY2 among the genes that are said to be expressed in the primitive endoderm has been checked, the expression amount in the iPS cell of the low-producing clone group tends to be higher than the expression amount in the iPS cell of the high-producing clone group. Table 4 shows the Fold Changes (Low Avg / High Avg) and the P-values of NODAL, CER1, CXCR4, LEFTY1, and LEFTY2.

**[Table 4]**

| Gene name | Fold Change (Low Avg/High Avg) | P-val |
|---|---|---|
| NODAL | 7.69 | 4.99E-08 |
| CER1 | 6.35 | 2.55E-07 |
| CXCR4 | 2.00 | 4.65E-05 |
| LEFTY1 | 1.87 | 2.00E-04 |
| LEFTY2 | 1.59 | 1.10E-03 |

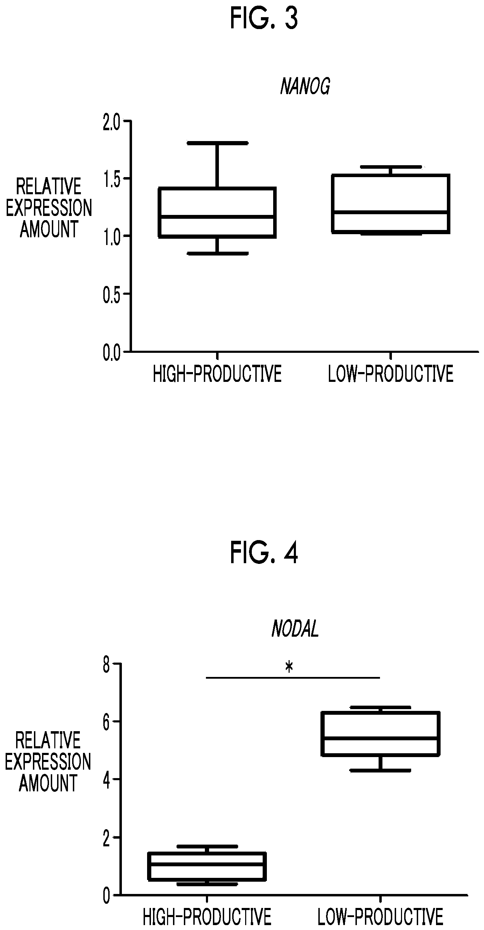

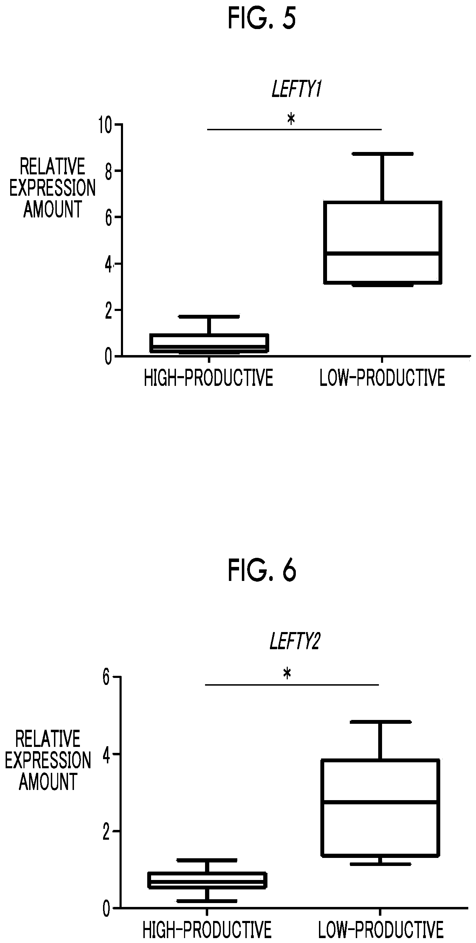

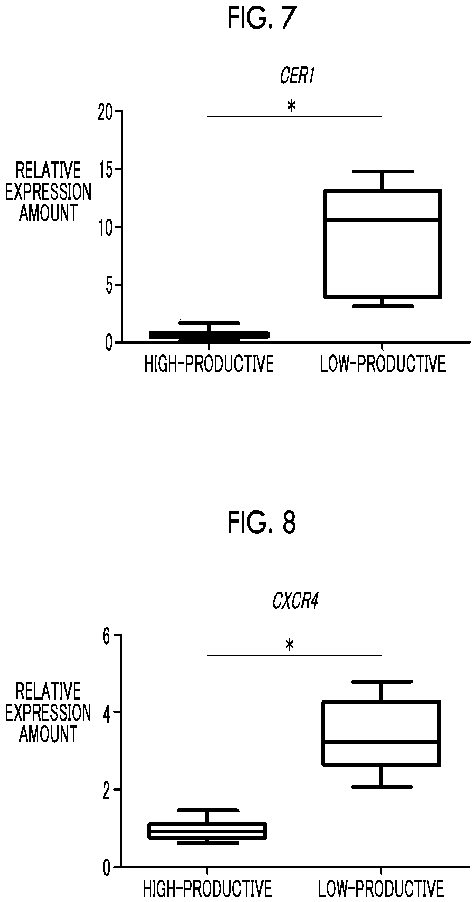

In order to confirm the above in more detail, a part of the RNA obtained from a part of cells sampled at the time of the expansion culture of the iPS cells was subjected to the reverse transcription into cDNA using a High capacity RNA-to-cDNA Kit (Thermo Fisher Scientific, Inc.), and a TaqMan (registered trade name) Gene Expression Assay (Thermo Fisher Scientific, Inc.) was used to quantify the expression amount of each gene by the ΔΔCT method with reference to the cDNA derived from the clone A. Table 5 shows the ID of the TaqMan (registered trade name) Probe used, and Table 6 shows the results obtained by evaluating the expression amount of each gene by the TaqMan (registered trade name) Probe. The results of the evaluation of each gene expression amount by the TaqMan (registered trade name) Probe are divided into the high-producing clone group and the low-producing clone group, and the results summarized in the box plots are shown in Figs. 3 to 8. It was confirmed that the expression amount of the pluripotency marker NANOG is not significantly different between the two groups, whereas the genes (NODAL, LEFTY1, LEFTY2, CER1, and CXCR4) that are said to be expressed in the primitive endoderm are significantly expressed high in the low-producing clones (* in the figure indicates P-value < 0.01).

From the above results, it was shown that iPS cell clones having relatively low myocardial cell productivity tend to have the high expression amount of the primitive endodermal lineage genes (the genes expressed by the primitive endoderm) in common.

**[Table 5]**

| Gene name | TaqMan (registered trade name) Gene Expression Assay ID |
|---|---|
| NANOG | Hs02387400_g1 |
| NODAL | Hs00415443_m1 |
| LEFTY1 | Hs00764128_s1 |
| LEFTY2 | Hs00745761_s1 |
| CER1 | Hs00193796_m1 |
| CXCR4 | Hs00607978_s1 |

**[Table 6]**

| Clone name | Myocardial cell productivity | NANOG | NODAL | LEFTY1 | LEFTY2 | CER1 | CXCR4 |
|---|---|---|---|---|---|---|---|
| A | High-producing group | 1.00 | 1.00 | 1.00 | 1.00 | 1.00 | 1.00 |
| B | | 1.13 | 0.47 | 0.16 | 0.19 | 0.15 | 0.77 |
| E | | 1.17 | 1.18 | 0.36 | 0.69 | 0.71 | 0.75 |
| F | | 1.17 | 1.02 | 1.71 | 1.25 | 0.84 | 0.90 |
| G | | 1.25 | 1.62 | 0.52 | 0.87 | 1.66 | 0.82 |
| I | | 0.97 | 1.12 | 0.22 | 0.68 | 0.81 | 0.62 |
| J | | 1.81 | 0.57 | 0.18 | 0.56 | 0.34 | 0.92 |
| K | | 1.47 | 0.39 | 0.34 | 0.56 | 0.48 | 1.46 |
| L | | 0.85 | 1.40 | 0.45 | 0.53 | 0.70 | 1.20 |
| M | | 1.40 | 1.69 | 0.88 | 0.79 | 0.72 | 1.07 |
| C | Low-producing group | 1.02 | 6.14 | 8.73 | 4.83 | 11.44 | 4.79 |
| D | | 1.05 | 4.31 | 4.54 | 2.84 | 10.62 | 3.75 |
| H | | 1.21 | 6.48 | 4.43 | 2.76 | 14.82 | 3.22 |
| N | | 1.46 | 5.37 | 3.29 | 1.59 | 4.77 | 3.19 |

### <Example 5>

### Differentiation induction into ectodermal cell

Among the 14 iPS cell clone strains, clones B, H, I, and N were selected and subjected to the expansion culture in the same manner as in Example 1. After proliferating iPS cells until the confluency reached about 80%, the cells were detached to be single cells with TrypLE (registered trade name) and collected, and the cell concentration was adjusted to 1 × 10⁶ cells/mL in a STEMdiff (registered trade name) Trilineage Ectoderm Medium (STEMCELL Technologies Inc.) containing, in terms of final concentration, 10 µmol/L Y-27632 (Fujifilm Wako Pure Chemical Industries, Ltd.) and plated on a 6-well plate coated with Matrigel (registered trade name). From the next day, the medium was changed daily with a STEMdiff (registered trade name) Trilineage Ectoderm Medium and cultured for 7 days after plating.

### <Example 6>

Correlation analysis between gene expression of iPS cell and ectodermal cell differentiation rate

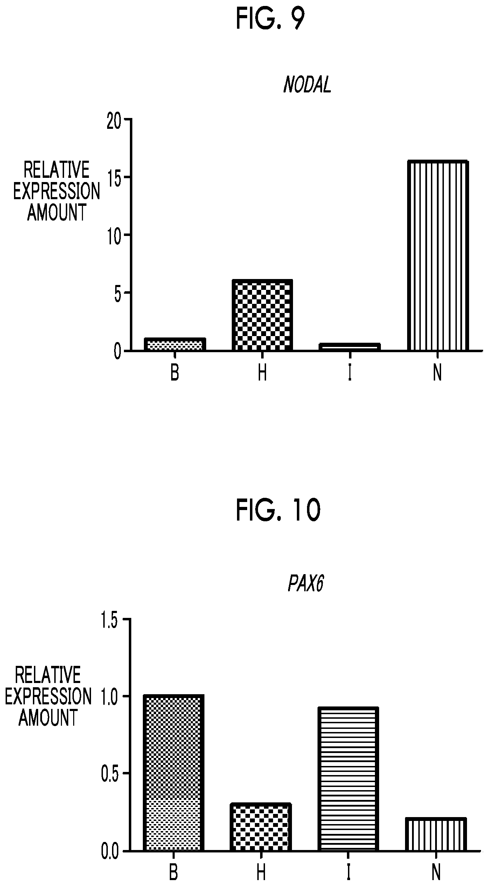

After culturing the cells for 7 days after the plating, according to the method described in Example 5, RNA was collected using an RNeasy Plus Mini Kit. The RNA was subjected to the reverse transcription into cDNA in the same manner as in Example 4, and the expression amount of the PAX6 gene, which is ectoderm marker gene, was analyzed using a TaqMan (registered trade name) Gene Expression Assay (ID: Hs01088114_m1). In addition, RNA after the expansion culture of each iPS clone (0 days after plating) was also collected in the same manner, and the expression amount of NODAL, which is a primitive endoderm gene, was measured (ID: Hs00415443_m1). The expression amount was quantified by the ΔΔCT method with reference to the clone B. The results are shown in Table 7 and Figs. 9 and 10. It was shown that both the iPS clones (the clones H and N) having high expression of the primitive endoderm gene had a relatively low PAX6 expression amount, that is, a low ectodermal differentiation efficiency, after the differentiation induction into ectoderm.

**[Table 7]**

| Clone name | NODAL expression before differentiation induction | PAX6 expression after differentiation induction into ectoderm |
|---|---|---|
| B | 1.00 | 1.00 |
| H | 6.02 | 0.30 |
| I | 0.54 | 0.92 |
| N | 16.31 | 0.21 |

## Claims

1. A producing method for a pluripotent stem cell capable of differentiating into a specific cell, the method comprising:
(i) a step of measuring an expression level of a gene, which is expressed by a primitive endodermal cell, in a pluripotent stem cell as a sample; and
(ii) a step of comparing the measured expression level of the gene, which is expressed by the primitive endodermal cell, with an expression level of the gene in a pluripotent stem cell known to have high differentiation potency into the specific cell, and selecting a pluripotent stem cell in which the measured expression level of the gene is less than one time relative to the expression level in the pluripotent stem cell known to have high differentiation potency into the specific cell, and acquiring a pluripotent stem cell capable of differentiating into the specific cell,
wherein the gene expressed by the primitive endodermal cell is at least one gene selected from the group consisting of CER1, LEFTY1, LEFTY2, NODAL, and CXCR4, and
wherein the specific cell is a differentiated cell derived from mesoderm or ectoderm.

2. The method according to claim 1,
wherein the specific cell is a myocardial cell or a nerve cell.

3. The method according to claim 1 or 2,
wherein the pluripotent stem cell is a human derived cell.

4. The method according to any one of claims 1 to 3,
wherein the pluripotent stem cell as the sample is obtained by clonally culturing and proliferating a pluripotent stem cell.

5. A producing method for a differentiated cell, comprising:
a step of obtaining a pluripotent stem cell capable of differentiating into a specific cell, by the method according to any one of claims 1 to 4; and
a step of inducing differentiation of the pluripotent stem cell capable of differentiating into a specific cell, obtained in the above step.

6. The method according to claim 5,
wherein the differentiated cell is a cell derived from mesoderm or ectoderm.

7. A method for quality evaluation of a pluripotent stem cell, the method comprising:
(a) a step of measuring an expression level of a gene, which is expressed by a primitive endodermal cell, in a pluripotent stem cell as a sample;
(b) a step of comparing the measured expression level of the gene, which is expressed by the primitive endodermal cell, with an expression level of the gene in a pluripotent stem cell known to have high differentiation potency into the specific cell; and
(c) a step of determining that a pluripotent stem cell in which the measured expression level of the gene is less than one time relative to the expression level in the pluripotent stem cell known to have high differentiation potency into the specific cell is excellent,
wherein the gene expressed by the primitive endodermal cell is at least one gene selected from the group consisting of CER1, LEFTY1, LEFTY2, NODAL, and CXCR4, and
wherein the specific cell is a differentiated cell derived from mesoderm or ectoderm.

8. A method for screening a pluripotent stem cell capable of differentiating into a specific cell, the method comprising the following steps:
(i) a step of measuring an expression level of a gene, which is expressed by a primitive endodermal cell, in a pluripotent stem cell as a sample; and
(ii) a step of comparing the measured expression level of the gene, which is expressed by the primitive endodermal cell, with an expression level of the gene in a pluripotent stem cell known to have high differentiation potency into the specific cell, and selecting a pluripotent stem cell in which the measured expression level of the gene is less than one time relative to the expression level in the pluripotent stem cell known to have high differentiation potency into the specific cell,
wherein the gene expressed by the primitive endodermal cell is at least one gene selected from the group consisting of CER1, LEFTY1, LEFTY2, NODAL, and CXCR4, and
wherein the specific cell is a differentiated cell derived from mesoderm or ectoderm.

## Patentansprüche

1. Herstellungsverfahren für eine pluripotente Stammzelle, die fähig ist, sich in eine spezifische Zelle zu differenzieren, das Verfahren umfassend:
(i) einen Schritt des Messens eines Expressionsniveaus eines Gens, das von einer primitiven endodermalen Zelle exprimiert wird, in einer pluripotenten Stammzelle als eine Probe; und
(ii) einen Schritt des Vergleichens des gemessenen Expressionsniveaus des Gens, das von der primitiven endodermalen Zelle exprimiert wird, mit einem Expressionsniveau des Gens in einer pluripotenten Stammzelle, die bekanntlich eine hohe Differenzierungspotenz in die spezifische Zelle hat, und Auswählen einer pluripotenten Stammzelle, in der das gemessene Expressionsniveau des Gens weniger als ein Mal relativ zum Expressionsniveau in der pluripotenten Stammzelle ist, die bekanntlich eine hohe Differenzierungspotenz in die spezifische Zelle hat, und Gewinnen einer pluripotenten Stammzelle, die fähig ist, sich in die spezifische Zelle zu differenzieren,
wobei das Gen, das von der primitiven endodermalen Zelle exprimiert wird, mindestens ein Gen, ausgewählt aus der Gruppe bestehend aus CER1, LEFTY1, LEFTY2, NODAL und CXCR4, ist, und
wobei die spezifische Zelle eine differenzierte Zelle ist, die vom Mesoderm oder Ektoderm abgeleitet ist.

2. Verfahren gemäß Anspruch 1,
wobei die spezifische Zelle eine Herzmuskelzelle oder eine Nervenzelle ist.

3. Verfahren gemäß Anspruch 1 oder 2,
wobei die pluripotente Stammzelle eine vom Menschen abgeleitete Zelle ist.

4. Verfahren gemäß irgendeinem der Ansprüche 1 bis 3,
wobei die pluripotente Stammzelle als die Probe durch klonales Kultivieren und Proliferieren einer pluripotenten Stammzelle erhalten wird.

5. Herstellungsverfahren für eine differenzierte Zelle, umfassend:
einen Schritt des Erhaltens einer pluripotenten Stammzelle, die fähig ist, sich in eine spezifische Zelle zu differenzieren, durch das Verfahren gemäß irgendeinem der Ansprüche 1 bis 4; und
einen Schritt des Induzierens der Differenzierung der pluripotenten Stammzelle, die fähig ist, sich in eine spezifische Zelle zu differenzieren, die in dem vorstehenden Schritt erhalten wurde.

6. Verfahren gemäß Anspruch 5,
wobei die differenzierte Zelle eine vom Mesoderm oder Ektoderm abgeleitete Zelle ist.

7. Verfahren zur Qualitätsbewertung einer pluripotenten Stammzelle, das Verfahren umfassend:
(a) einen Schritt des Messens eines Expressionsniveaus eines Gens, das von einer primitiven endodermalen Zelle exprimiert wird, in einer pluripotenten Stammzelle als eine Probe;
(b) einen Schritt des Vergleichens des gemessenen Expressionsniveaus des Gens, das von der primitiven endodermalen Zelle exprimiert wird, mit einem Expressionsniveau des Gens in einer pluripotenten Stammzelle, die bekanntlich eine hohe Differenzierungspotenz in die spezifische Zelle hat; und
(c) einen Schritt des Bestimmens, dass eine pluripotente Stammzelle, in der das gemessene Expressionsniveau des Gens weniger als ein Mal relativ zum Expressionsniveau in der pluripotenten Stammzelle ist, die bekanntlich eine hohe Differenzierungspotenz in die spezifische Zelle hat, exzellent ist,
wobei das Gen, das von der primitiven endodermalen Zelle exprimiert wird, mindestens ein Gen, ausgewählt aus der Gruppe bestehend aus CER1, LEFTY1, LEFTY2, NODAL und CXCR4, ist, und
wobei die spezifische Zelle eine differenzierte Zelle ist, die vom Mesoderm oder Ektoderm abgeleitet ist.

8. Auswahlverfahren einer pluripotenten Stammzelle, die fähig ist, sich in eine spezifische Zelle zu differenzieren, das Verfahren umfassend die folgenden Schritte:
(i) einen Schritt des Messens eines Expressionsniveaus eines Gens, das von einer primitiven endodermalen Zelle exprimiert wird, in einer pluripotenten Stammzelle als eine Probe; und
(ii) einen Schritt des Vergleichens des gemessenen Expressionsniveaus des Gens, das von der primitiven endodermalen Zelle exprimiert wird, mit einem Expressionsniveau des Gens in einer pluripotenten Stammzelle, die bekanntlich eine hohe Differenzierungspotenz in die spezifische Zelle hat, und des Auswählens einer pluripotenten Stammzelle, in der das gemessene Expressionsniveau des Gens weniger als ein Mal relativ zum Expressionsniveau in der pluripotenten Stammzelle ist, die bekanntlich eine hohe Differenzierungspotenz in die spezifische Zelle hat,
wobei das Gen, das von der primitiven endodermalen Zelle exprimiert wird, mindestens ein Gen, ausgewählt aus der Gruppe bestehend aus CER1, LEFTY1, LEFTY2, NODAL und CXCR4, ist, und
wobei die spezifische Zelle eine differenzierte Zelle ist, die vom Mesoderm oder Ektoderm abgeleitet ist.

## Revendications

1. Procédé de production d'une cellule souche pluripotente capable de se différencier en une cellule spécifique, le procédé comprenant :
(i) une étape de mesure d'un niveau d'expression d'un gène, qui est exprimé par une cellule endodermique primitive, dans une cellule souche pluripotente en tant qu'échantillon ; et
(ii) une étape de comparaison du niveau d'expression mesuré du gène, qui est exprimé par la cellule endodermique primitive, à un niveau d'expression du gène dans une cellule souche pluripotente connue pour présenter un pouvoir de différenciation élevé en la cellule spécifique, et une sélection d'une cellule souche pluripotente dans laquelle le niveau d'expression mesuré du gène est inférieur à une fois par rapport au niveau d'expression dans la cellule souche pluripotente connue pour présenter un pouvoir de différenciation élevé en la cellule spécifique, et une acquisition d'une cellule souche pluripotente capable de se différencier en la cellule spécifique, dans lequel le gène exprimé par la cellule endodermique primitive est au moins un gène sélectionné parmi le groupe consistant en CER1, LEFTY1, LEFTY2, NODAL et CXCR4, et dans lequel la cellule spécifique est une cellule différenciée dérivée de mésoderme ou d'ectoderme.

2. Procédé selon la revendication 1,
dans lequel la cellule spécifique est une cellule myocardique ou une cellule nerveuse.

3. Procédé selon la revendication 1 ou la revendication 2,
dans lequel la cellule souche pluripotente est une cellule dérivée humaine.

4. Procédé selon l'une quelconque des revendications 1 à 3,
dans lequel la cellule souche pluripotente en tant qu'échantillon est obtenue par culture clonale et prolifération d'une cellule souche pluripotente.

5. Procédé de production d'une cellule différenciée, comprenant :
une étape d'obtention d'une cellule souche pluripotente capable de se différencier en une cellule spécifique, par le procédé selon l'une quelconque des revendications 1 à 4 ; et
une étape d'induction d'une différenciation de la cellule souche pluripotente capable de se différencier en une cellule spécifique, obtenue dans l'étape ci-dessus.

6. Procédé selon la revendication 5,
dans lequel la cellule différenciée est une cellule dérivée de mésoderme ou d'ectoderme.

7. Procédé d'évaluation de la qualité d'une cellule souche pluripotente, le procédé comprenant :
(a) une étape de mesure d'un niveau d'expression d'un gène, qui est exprimé par une cellule endodermique primitive, dans une cellule souche pluripotente en tant qu'échantillon ;
(b) une étape de comparaison du niveau d'expression mesuré du gène, qui est exprimé par la cellule endodermique primitive, à un niveau d'expression du gène dans une cellule souche pluripotente connue pour présenter un pouvoir de différenciation élevé en la cellule spécifique ; et
(c) une étape de détermination établissant qu'une cellule souche pluripotente dans laquelle le niveau d'expression mesuré du gène est inférieur à une fois par rapport au niveau d'expression dans la cellule souche pluripotente connue pour présenter un pouvoir de différenciation élevé en la cellule spécifique est excellent, dans lequel le gène exprimé par la cellule endodermique primitive est au moins un gène sélectionné parmi le groupe consistant en CER1, LEFTY1, LEFTY2, NODAL et CXCR4, et dans lequel la cellule spécifique est une cellule différenciée dérivée de mésoderme ou d'ectoderme.

8. Procédé de criblage d'une cellule souche pluripotente capable de se différencier en une cellule spécifique, le procédé comprenant les étapes suivantes :
(i) une étape de mesure d'un niveau d'expression d'un gène, qui est exprimé par une cellule endodermique primitive, dans une cellule souche pluripotente en tant qu'échantillon ; et
(ii) une étape de comparaison du niveau d'expression mesuré du gène, qui est exprimé par la cellule endodermique primitive, à un niveau d'expression du gène dans une cellule souche pluripotente connue pour présenter un pouvoir de différenciation élevé en la cellule spécifique, et une sélection d'une cellule souche pluripotente dans laquelle le niveau d'expression mesuré du gène est inférieur à une fois par rapport au niveau d'expression dans la cellule souche pluripotente connue pour présenter un pouvoir de différenciation élevé en la cellule spécifique,
dans lequel le gène exprimé par la cellule endodermique primitive est au moins un gène sélectionné parmi le groupe consistant en CER1, LEFTY1, LEFTY2, NODAL et CXCR4, et dans lequel la cellule spécifique est une cellule différenciée dérivée de mésoderme ou d'ectoderme.
